(19) Europäisches Patentamt
European Patent Office
Office européen des brevets

(11) EP 3 982 112 A1

(12) # EUROPEAN PATENT APPLICATION
published in accordance with Art. 153(4) EPC

(43) Date of publication:
**13.04.2022 Bulletin 2022/15**

(21) Application number: **20817756.8**

(22) Date of filing: **02.06.2020**

(51) International Patent Classification (IPC):
***G01N 21/49*** *(2006.01)* ***A61B 5/1455*** *(2006.01)*

(52) Cooperative Patent Classification (CPC):
**A61B 5/1455; G01N 21/49**

(86) International application number:
**PCT/JP2020/021723**

(87) International publication number:
**WO 2020/246455 (10.12.2020 Gazette 2020/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.06.2019 JP 2019104467**

(71) Applicant: **Medical Photonics Co., Ltd.**
**Sapporo-shi, Hokkaido 001-0021 (JP)**

(72) Inventors:
- **SHIMIZU, Koichi**
**Kitakyushu-shi, Fukuoka 808-0135 (JP)**
- **IINAGA, Kazuya**
**Sapporo-shi, Hokkaido 001-0021 (JP)**

(74) Representative: **Vossius & Partner Patentanwälte Rechtsanwälte mbB**
**Siebertstraße 3**
**81675 München (DE)**

(54) # NON-INVASIVE MEASURING DEVICE, METHOD, AND PROGRAM

(57) [Problem] To realize high-precision non-invasive measurement.

[Solution] This non-invasive measuring device is provided with: an irradiation unit which is for irradiating a specific position on a test subject with light; a light intensity detection unit which is disposed at a position on a blood vessel of the test subject and which is for detecting a first light intensity of light emitted from a position, on the test subject, located at a prescribed distance from the position irradiated with light by the irradiation unit; a light intensity detection unit which is disposed at a position not located on a blood vessel of the test subject and which is for detecting a second light intensity of light emitted from a position, on the test subject, located at a prescribed distance from the position irradiated with light by the irradiation unit; and a control unit which calculates a third light intensity of the blood in a blood vessel from the first light intensity and the second light intensity, a correct attenuation coefficient from a temporary attenuation coefficient obtained from the third light intensity by means of a calibration function, and an equivalent scattering coefficient (turbidity coefficient of blood, etc.) and an absorption coefficient (spectroscopic information of blood, etc.) separately from the attenuation coefficient through measurement of multiple wavelengths.

FIG. 1

1
4
A
B
CONTROL UNIT
2
21
31a
31b
31c
31d
31e
31f
$\rho_1$
$\rho_2$
$\rho_3$

EP 3 982 112 A1

## Description

Technical Field

The present invention relates to a noninvasive measuring device, method and program.

Background Art

Non-Patent Literature 1 discloses the principle of measurement of the blood turbidity (reflecting in-blood lipid concentration) from the spatial distribution of reflectance R(ρ) (intensity of backscattered light produced at body surface when light is incident on single point on body surface). The reflectance R(ρ) for a transmitter-receiver interval ρ is given by Numerical Formula 1.

[Numerical Formula 1]

$$R(\rho) = \frac{S_0}{2\pi\mu_s'}\left(\mu_{eff} + \frac{1}{\rho}\right)\frac{1}{\rho^2}e^{-\mu_{eff}\rho}$$

Citation List

Patent Literature

Patent Literature 1: Japanese Patent No. 6,241,853

Non-Patent Literature

Non-Patent Literature 1: SHIMIZU Koichi, Analysis of Light Propagation in Biological Tissue, Japanese Journal of Optics, Vol. 41, No. 8, pp. 414-423, 2012

Summary of Invention

Technical Problem

The approach described above, however, has the following three primary problems, which force the blood turbidity measurement to be of low accuracy. (1) The reflectance measured by the approach described above does not allow measurement of pure blood turbidity because the reflectance contains not only backscattered light from the blood but backscattered light from living body tissue excluding the blood. (2) The principle described in Non-Patent Literature 1, on which the approach described above is based, is derived from diffusion approximation, and the principle works well with living body tissue excluding blood but cannot be applied to blood for which the approximation condition does not hold true. (3) The approach described above provides an attenuation coefficient $\mu_{eff}$, and the value of an absorption coefficient $\mu_a$ is required to accurately determine an equivalent scattering coefficient $\mu_s$', which represents blood turbidity. That is, to accurately measure the blood turbidity, the absorption coefficient $\mu_a$ also needs to be measured at the same time.

The present invention has been made to solve the three points (1) to (3) described above, and an object of the present invention is to achieve highly accurate, noninvasive measurement.

Solution to Problem

A noninvasive measuring device according to the present invention includes a light radiator that radiates light to a predetermined position on a subject, optical intensity detectors that are disposed in positions above a blood vessel of the subject and detect a first optical intensity of light emitted from the subject in positions separate at predetermined intervals from a position irradiated with the light from the light radiator, optical intensity detectors that are disposed in positions not above the blood vessel of the subject and detect a second optical intensity of light emitted from the subject in positions separate at predetermined intervals from the position irradiated with the light from the light radiator, and a control unit that calculates a third optical intensity from blood in the blood vessel by using the first optical intensity and the second optical intensity.

A noninvasive measuring program according to the present invention causes a computer in a device including a light radiator that radiates light to a predetermined position on a subject, optical intensity detectors that are disposed in positions above a blood vessel of the subject and detect a first optical intensity of light emitted from the subject in positions separate at predetermined intervals from a position irradiated with the light from the light radiator, and optical intensity detectors that are disposed in positions not above the blood vessel of the subject and detect a second optical intensity of light emitted from the subject in positions separate at predetermined intervals from the position irradiated with the light from the light radiator to carry out the process of calculating a third optical intensity from blood in the blood vessel by using the first optical intensity and the second optical intensity.

A noninvasive measuring method according to the present invention includes radiating light to a predetermined position on a subject, detecting a first optical intensity of light emitted from the subject in positions on a blood vessel of the subject and separate at predetermined intervals from a position irradiated with the light, detecting a second optical intensity of light emitted from the subject in positions not on the blood vessel of the subject and separate at predetermined intervals from the position irradiated with the light, and calculating a third optical intensity from blood in the blood vessel by using the first optical intensity and the second optical intensity.

Brief Description of Drawings

[Figure 1] Figure 1 shows the configuration of a noninvasive measuring device according to an embodiment.
[Figure 2] Figure 2 shows the arrangement of an optical intensity detector.
[Figure 3] Figure 3 shows that light is scattered by in-blood lipids.
[Figure 4] Figure 4 is a block diagram of a control unit.
[Figure 5] Figure 5 shows Numerical Formula 5 graphed versus a transmitter-receiver interval $\rho$.
[Figure 6] Figure 6 is a flowchart of noninvasive measurement according to the embodiment.

Description of Embodiment

A noninvasive measuring device, program, and method according to an embodiment will be described below in detail with reference to the drawings.

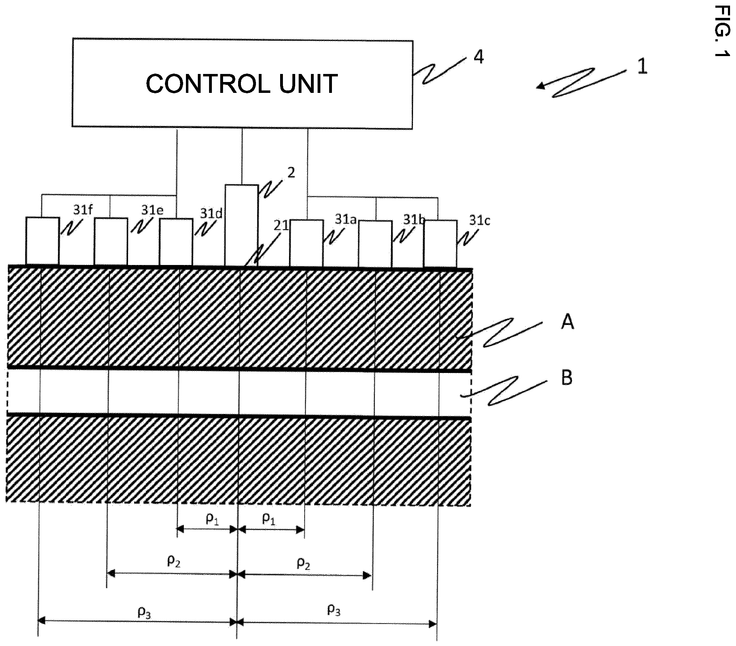

Figure 1 shows the configuration of the noninvasive measuring device according to the embodiment. Figure 1 does not show a second optical intensity detector group 32, in which second optical intensity detectors 32a to 32f are aligned in a row.

A noninvasive measuring device 1 includes a light radiator 2, an optical intensity detector 3, and a control unit 4, as shown in Figure 1. In the drawings, reference character A represents a living body, and reference character B represents a blood vessel in the living body.

The light radiator 2 includes a light source that radiates light to a predetermined radiation position 21 on the predetermined site of the living body from a point outside the living body toward the interior of the living body. The light radiator 2 may be controlled by the control unit 4 in terms of the wavelength and radiation intensity of the light to be radiated.

The light radiator 2 in the embodiment can arbitrarily adjust the length of time, for example, for which the light is radiated continuously or in the form of pulsed light.

The light radiator 2 may be formed of a light source having a fixed wavelength. The light radiator 2 may be the combination of a plurality of light sources that output light fluxes having different wavelengths or may output the mixture of light fluxes having a plurality of wavelengths. The light radiator 2 is, for example, a fluorescent lamp, an LED, a laser, an incandescent lamp, an HID, or a halogen lamp. The illuminance of the light from the light radiator 2 may be controlled by the control unit 4.

The light radiator 2 is an LED (light emitting diode) in the embodiment. The light radiator 2 in the embodiment radiates light having a first wavelength ($\lambda_1$) and light having a second wavelength ($\lambda_2$), which differs from the first wavelength. The number of light radiator 21 may be one, or a plurality of light radiators 21 may, for example, form a first optical intensity detector group 31 and a second optical intensity detector group 32, which will be described later.

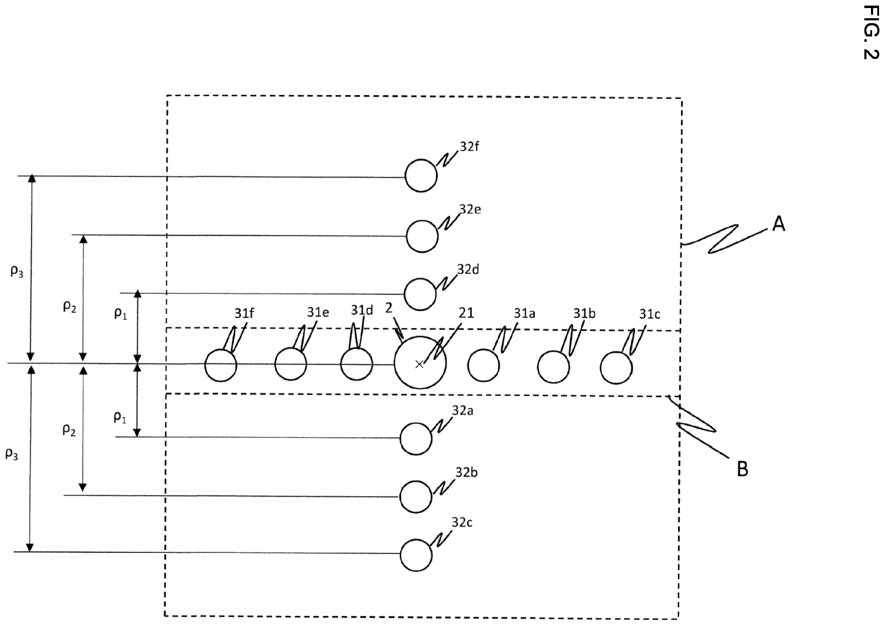

Figure 2 shows the arrangement of the optical intensity detector 3. The dotted lines in the figures indicate a blood vessel B in a living body A. The optical intensity detector 3 includes detectors arranged in the form of a cross on the surface of the living body (on the skin), as shown in Figure 2. The optical intensity detector 3 receives light emitted from the living body toward the space outside the living body and detects the optical intensity of the light. The optical intensity detector 3 may be formed of photodiodes, a CCD, or a CMOS device.

The optical intensity detector 3 includes the first optical intensity detector group 31, in which first optical intensity detectors 31a to 31f located at the living body surface above the blood vessel B in the living body A and aligned in a row in the direction along the blood vessel, and the second optical intensity detector group 32, in which second optical intensity detectors 32a to 32f aligned in a row in the direction perpendicular to the first optical intensity detector group 31 (that is, in positions separate away from blood vessel).

The first optical intensity detectors 31a to 31f are disposed at different distances from the center of the radiation position 21, to which the light radiator 2 radiates the light. In the embodiment, the first optical intensity detectors 31a to 31f are arranged in a single plane along a straight line at predetermined intervals from the radiation position 21, as shown in Figure 1.

The distance from the radiation position 21 to the first optical intensity detectors 31a and 31d is $\rho_1$, the distance from the radiation position 21 to the first optical intensity detectors 31b and 31e is $\rho_2$, and the distance from the radiation position 21 to the first optical intensity detectors 31c and 31f is $\rho_3$.

The second optical intensity detectors 32a to 32f are disposed at different distances from the center of the radiation position 21, to which the light radiator 2 radiates the light. In the embodiment, the second optical intensity detectors 32a to 32f are arranged in a single plane along a straight line at predetermined intervals from the radiation position 21, as shown in Figure 2.

The distance from the radiation position 21 to the second optical intensity detectors 32a and 32d is $\rho_1$, the distance from the radiation position 21 to the second optical intensity detectors 32b and 32e is $\rho_2$, and the distance from the radiation position 21 to the second optical intensity detectors 32c and 32f is $\rho_3$.

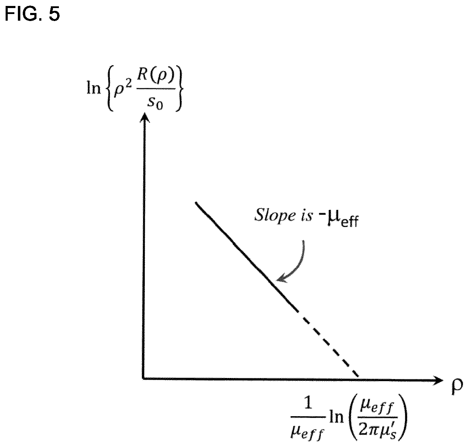

In the embodiment, the number of first optical intensity detectors 31 and the number of second optical intensity detectors are each set at six, but not necessarily. In the embodiment, an attenuation coefficient $\mu_{eff}$ is estimated from the gradient of the graph in Figure 5. Therefore, at least two detectors only need to be present on each side of the light incident point, and the greater the number of detectors, the more accurate the estimation of the attenuation coefficient $\mu_{eff}$.

In related art, in which photodetectors are disposed at the body surface along a vein under observation, the backscattered light scattered through the blood is measured as much as possible, and the measured value is used as the blood reflectance. The actually measured backscattered light, however, contains not only the backscattered light from the blood but not a little amount of backscattered light from the living body tissue around the blood vessel. In view of the fact described above, the reflectance containing as little backscattered light as possible from the vessel is measured around the blood vessel, and the contribution of the measured reflectance to the reflectance is excluded. Pure reflectance from the blood in the vessel can thus be determined.

In the embodiment, the second optical intensity detectors 32a to 32f are disposed in the direction perpendicular to the direction in which the vessel extends, as a method for measuring the "reflectance containing as little backscattered light as possible." The arrangement of the second optical intensity detectors 32a to 32f is not limited to that described above, and the light source and the second optical intensity detectors 32a to 32f may be disposed along a line shifted from the extending blood vessel in parallel thereto.

The light received by the optical intensity detector 3 is converted into photocurrent, which is processed by the control unit 4.

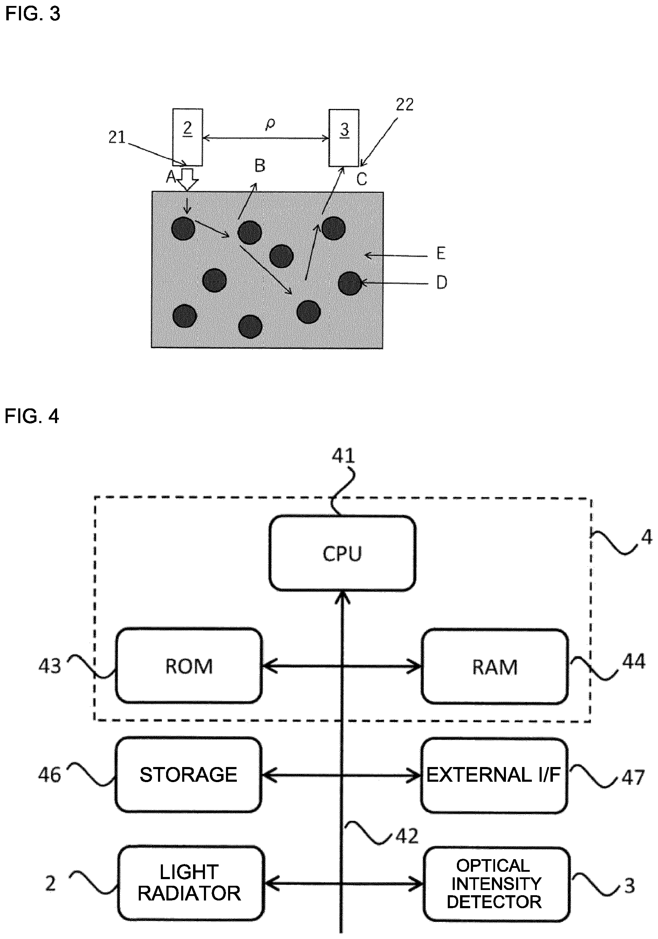

A predetermined distance $\rho$ is set between the radiation position 21, where the living body is irradiated with the light, and a detection position 31, where the intensity of the light emitted from the blood (E in Figure 3) in the living body is detected, as shown in Figure 3. The thus set predetermined distance $\rho$ suppresses the influence of the light directly emitted from the living body (B in Figure 3), which is the radiated light (A in Figure 3) reflected off the surface of the living body and scatterers in the vicinity of the surface. After the radiated light reaches the depth where the blood is present, the light is scattered by the lipids in the blood (D in the figure), and the backscattered light is detected at the surface of the living body.

In the case where a plurality of first optical intensity detectors and a plurality of second optical intensity detectors are provided, the detectors are not necessarily arranged linearly as long as the detectors are disposed at different distances roughly around the radiation position 21, and the arrangement of the detectors can be selected as appropriate from a circular arrangement, a wavy arrangement, a zigzag arrangement, and other arrangements. The first radiation-detection distance $\rho_1$, the second radiation-detection distance $\rho_2$, and the third radiation-detection distance $\rho_3$, which are each the distance from the radiation position 21 to the corresponding one of the first and second optical intensity detectors, the interval between the first optical intensity detectors, the interval between the second optical intensity detectors are not each limited to a fixed distance, and the optical intensity detectors may instead be continuously arranged.

Furthermore, in the embodiment, the first optical intensity detector group 31, in which the first optical intensity detectors 31a to 31f are aligned in a row, and the second optical intensity detector group 32, in which the second optical intensity detectors 32a to 32f are aligned in a row, are perpendicular to each other, but not necessarily, and the first optical intensity detector group 31 and the second optical intensity detector group 32 only needs to be so configured that the first optical intensity detectors 31a to 31f are above the blood vessel B and the second optical intensity detectors 32a to 32f are away from the blood vessel B.

For example, when the first optical intensity detectors 31a to 31f are above the vessel B and the second optical intensity detectors 32a to 32f are away from the vessel B, the first optical intensity detector group 31, in which the first optical intensity detectors 31a to 31f are aligned in a row, and the second optical intensity detector groups 32, in which the second optical intensity detectors 32a to 32f are aligned in a row, can be shifted from each other in parallel to each other.

The configuration of a control system of the noninvasive measuring device 1 will next be described. Figure 4 is a block diagram of the control unit of the noninvasive measuring device according to the embodiment. A CPU (central processing unit) 41, a ROM (read only memory) 43, a RAM (random access memory) 44, a storage 46, an external I/F (interface) 47, the light radiator 2, and the optical intensity detector 3 are connected to each other via a system bus 42. The CPU 41, the ROM 43, and the RAM 44 form the control unit (controller) 4.

The ROM 43 stores in advance a program executed by the CPU 41 and thresholds used by the CPU 41.

The RAM 44 has an area where the program executed by the CPU 41 is developed, a variety of memory areas, such as a work area where the program processes data, and other areas.

The storage 46 stores data, such as sensed and calculated optical intensities and $\mu_{eff}$. The storage 46 may be an internal memory that stores information in a nonvolatile manner, such as an HDD (hard disk drive), a flash memory, and an SSD (solid-state drive).

The external I/F 47 is an interface for communication with an external device, for example, a client terminal (PC). The external I/F 47 only needs to be an interface that performs data communication with an external device and may, for example, be an instrument (such as USB memory) locally connected to the external device or a network interface for communication via a network.

At the surface of the living body, the first optical intensity detectors 31a to 31f immediately above the blood vessel measure reflectance $R_{on}(\rho)$ and transmit the reflectance $R_{on}(\rho)$ to the control unit 4. The second optical intensity detectors 32a to 32f, which are separate from the blood vessel, measure reflectance $R_{off}(\rho)$ and send the reflectance $R_{off}(\rho)$ to the control unit 4.

Let $\alpha$**(p)** and $\beta$**(**$\rho$**)** be a contribution factor of blood reflectance $R_{blood}(\rho)$ to $R_{on}(\rho)$ and $R_{off}(\rho)$, respectively. The contribution factors $\alpha(\rho)$ and $\beta(\rho)$ are determined in advance by simulations or experiments. Furthermore, ratios $k_s$ and $k_a$ between the equivalent scattering coefficient and the absorption coefficient at two wavelengths to be used are also determined in advance from values in literatures or from experiments performed by the users.

The control unit 4 uses $R_{on}(\rho)$ measured by the first optical intensity detectors 31a to 31f and $R_{off}(\rho)$ measured by the second optical intensity detectors 32a to 32f to determine the blood reflectance $R_{blood}(\rho)$ excluding the contribution of living body tissue. The method for calculating the reflectance $R_{blood}(\rho)$ is as follows.

Numerical formula 2 shows that the reflectance $R_{on}$ provided from the first optical intensity detectors 31a to 31f, which are located immediately above the blood vessel and extending therealong, is formed of the blood reflectance $R_{blood}$, living body tissue reflectance $R_{tissue}$, and the proportions $\alpha$ and (1-$\alpha$) thereof.

[Numerical Formula 2]

$$R_{on}(\rho) = \alpha R_{blood}(\rho) + (1-\alpha) R_{tissue}(\rho)$$

Numerical formula 3 shows that the reflectance $R_{off}$ provided from the second optical intensity detectors 32a to 32f, which are disposed in the direction perpendicular to the direction in which the first optical intensity detectors 31a to 31f are disposed or are shifted in parallel to the first optical intensity detectors 31a to 31f, is formed of the blood reflectance $R_{blood,}$ the living body tissue reflectance $R_{tissue}$, and the proportions $\beta$ and (1-$\beta$) thereof.

[Numerical Formula 3]

$$R_{off}(\rho) = \beta R_{blood}(\rho) + (1-\beta) R_{tissue}(\rho)$$

The values $\alpha$ and $\beta$ (both are functions of transmitter-receiver interval $\rho$) are determined in advance by Monte Carlo simulations or experiments. Numerical Formula 4, which is derived from Numerical Formulae 3 and 4, allows determination of the blood reflectance $R_{blood}$ excluding the contribution of the living body tissue by using the measured $R_{on}$ and $R_{off}$.

[Numerical Formula 4]

$$R_{blood}(\rho) = \frac{(1-\beta)R_{on} - (1-\alpha)R_{off}}{\alpha - \beta}$$

The control unit 4 estimates the attenuation coefficient $\mu_{eff}$ based on the blood reflectance $R_{blood}$ (p) .

Patent Literature 1 discloses the principle of the measurement of the blood turbidity (reflecting in-blood lipid concentration) from the spatial distribution of the reflectance R(ρ) (intensity of backscattered light produced at body surface when light is incident on single point on body surface). The reflectance R(ρ) for the transmitter-receiver interval ρ is given by Numerical Formula 1 described above.

Therefore, R(ρ) provided for the transmitter-receiver interval ρ is measured and normalized by the amount of incident light $S_0$, and then the quantity on the left side of Numerical Formula 5 is calculated.

[Numerical Formula 5]

$$ln\left\{\rho^2 \frac{R(\rho)}{S_0}\right\} = -\mu_{eff}\rho + ln\left(\frac{\mu_{eff}}{2\pi\mu_s'}\right)$$

When Numerical Formula 5 is graphed versus the transmitter-receiver interval ρ, the graph is a straight line as shown in Figure 5, and the gradient of the graph is a negative value $-\mu_{eff}$ of the attenuation coefficient $\mu_{eff}$.

The relationship among the attenuation coefficient $\mu_{eff}$, the absorption coefficient $\mu_a$, and the equivalent scattering coefficient $\mu_s$' is given by Numerical Formula 6 below.

[Numerical Formula 6]

$$\mu_{eff} = \sqrt{3\mu_a(\mu_s' + \mu_a)}$$

Numerical Formula 6, when the absorption coefficient $\mu_a$ is known, determines the equivalent scattering coefficient $\mu_s$' from the attenuation coefficient $\mu_{eff}$. Since the blood turbidity is directly reflected in $\mu_s$', the blood turbidity can be evaluated by measuring the reflectance R(p) and determining the attenuation coefficient $\mu_{eff}$.

The control unit 4 calculates the left side $\ln\{\rho^2 R_{blood}(\rho)/S_0\}$ of Numerical Formula 5 from the light-transmission-light-reception interval ρ, the amount of incident light So, and the blood reflectance $R_{blood}(\rho)$. Figure 5 shows the graphed Numerical Formula with $\ln\{\rho^2 R_{blood}(\rho)/S_0\}$ representing the vertical axis and p representing the horizontal axis. Since the gradient of the graph in Figure 5 is the negative value of the attenuation coefficient $-\mu_{eff}$, the attenuation coefficient $\mu_{eff}$ can be estimated.

The control unit 4 determines a calibration coefficient k, which eliminates an approximation error of the diffusion approximation, by using the following calibration function. A correct, approximation-error-free attenuation coefficient $\mu_{eff}$ is provided by multiplying the attenuation coefficient $\mu_{eff,meas}$ provided in the process described above by the calibration coefficient k. That is, $\mu_{eff} = k\mu_{eff,meas}$.

A calibration curve function that eliminates an error resulting from the method based on the diffusion approximation is provided as shown by Numerical Formula 7 below.

[Numerical formula 7]

$$\mathrm{k} = \mathrm{a}e^{-\mathrm{b}\mu_{eff\,meas}} + c$$

When $\mu_{eff}$ ranges from 1.00 to 2.00, a, b, and c are given as follows: a = 55.7; b = 4.63; and c = 1.00. Symbol e represents the exponential function.

The correct attenuation coefficient $\mu_{eff}$ can thus be determined by first estimating a temporary attenuation coefficient $\mu_{eff,meas}$ from the blood reflectance $R_{blood}$ and then multiplying $\mu_{eff,meas}$ by the calibration coefficient k provided from the calibration function expressed by Numerical Formula 7.

The control unit 4 carries out the process described above by using light sources that output light having two wavelengths $\lambda_1$ and $\lambda_2$ to estimate attenuation coefficients $\mu_{eff}$ ($\lambda_1$)and $\mu_{eff}$ ($\lambda_2$). The control unit 4 uses the estimated $\mu_{eff}(\lambda_1)$ and $\mu_{eff}(\lambda_2)$ and an equivalent scattering coefficient ratio $k_s$ and an absorption coefficient ratio $k_a$, which are prepared in advance, to determine equivalent scattering coefficients $\mu_s'$ ($\lambda_1$) and $\mu_s'$ ($\lambda_2$) and absorption coefficients $\mu_a(\lambda_2)$ and $\mu_a(\lambda_2)$ at the two wavelengths.

The equivalent scattering coefficients $\mu_s'$ ($\lambda_1$) and $\mu_s'$ ($\lambda_2$) and the absorption coefficients $\mu_a$ ($\lambda_1$)and $\mu_a(\lambda_2)$ are separately determined from $\mu_{eff}$ ($\lambda_1$)and $\mu_{eff}$ ($\lambda_2$) provided in the process described above by measuring the reflectance at a plurality of wavelengths (two wavelengths $\lambda_1$ and $\lambda_2$) . The value $k_s$ is the ratio between the equivalent scattering coefficients at the two wavelengths, and the value $k_a$ is the ratio between the absorption coefficients at the two wavelengths, that is, $k_s=\mu_s'(\lambda_2)/\mu_s'(\lambda_1)$, and $k_a=\mu_a(\lambda_2)/\mu_a(\lambda_1)$ The $k_s$ and $k_a$ are determined in advance values in literatures or from experiments.

The control unit 4 separates and calculates $\mu_a(\lambda_1)$ by using Numerical Formula 8.

[Numerical Formula 8]

$$\mu_a(\lambda_1) = \sqrt{\frac{\mu_{eff}(\lambda_2) - k_a k_s \mu_{eff}(\lambda_1)}{3k_a(k_a - k_s)}}$$

The control unit 4 separates and calculates $\mu_s'$ ($\lambda_1$) by using Numerical Formula 9.

[Numerical Formula 9]

$$\mu_s'\ (\lambda_1) = \frac{\mu_{eff}{}^2\ (\lambda_1)}{3\mu_a(\lambda_1)} - \mu_a(\lambda_1)$$

The control unit 4 quantifies the degree of optical scattering, such as turbidity of a target object, from the equivalent scattering coefficients and identifies and quantifies the substances that form the target object from the absorption coefficients.

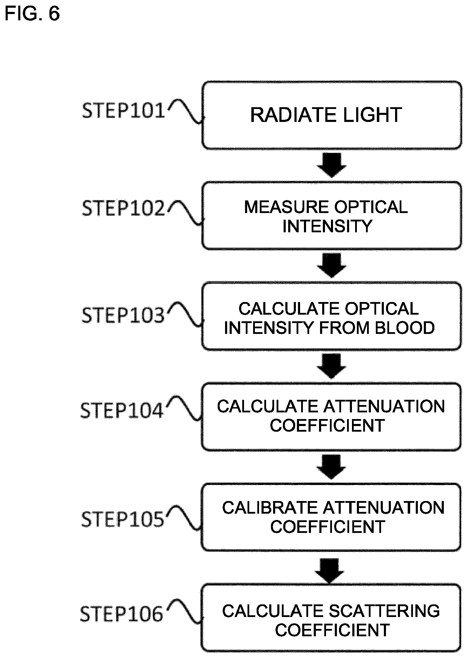

The noninvasive measuring device 1 having the configuration described above performs noninvasive measurement based on a program stored in advance. Figure 6 is a flowchart of the noninvasive measurement according to the embodiment. The program may be stored in a storage medium.

The light radiator 2 radiates radiation light toward a predetermined site of a living body from a point outside the living body toward the interior of the living body. The light radiator 2 may radiate light having a first wavelength ($\lambda_1$)and light having a second wavelength ($\lambda_2$), which differs from the first wavelength (step 101).

The first optical intensity detectors 31 measure the optical intensity (reflectance) in positions above a blood vessel of the living body and separate at predetermined intervals from a radiation position irradiated with the radiated light, and the second optical intensity detectors 32 measure the optical intensity (reflectance) in positions that are not above the blood vessel of the living body and separate at predetermined intervals from the radiation position irradiated with the radiated light (step 102).

The control unit 4 determines the blood reflectance $R_{blood}(\rho)$ excluding the contribution of the living body tissue by using $R_{on}(\rho)$ measured by the first optical intensity detectors 31 and $R_{off}(\rho)$ measured by the second optical intensity detectors 32 (step 103). The method for calculating the reflectance has been described above.

The control unit 4 estimates the attenuation coefficient $\mu_{eff}$ based on the blood reflectance $R_{blood}(\rho)$ (step 104). The method for calculating the attenuation coefficient $\mu_{eff}$ has been described above.

The control unit 4 provides the correct, approximation-error-free attenuation coefficient $\mu_{eff}$ by multiplying $\mu_{eff}$ estimated in the paragraph described above by the calibration coefficient (step 105).

The control unit 4 calculates the equivalent scattering coefficient $\mu_s'$ ($\lambda_1$)and absorption coefficient $\mu_a(\lambda_1)$ from

$\mu_{eff}(\lambda_1)$ and $(\mu_{eff}(\lambda_2)$ provided by carrying out the process described above using light sources that output light having the two wavelengths $\lambda_1$ and $\lambda_2$ (step 106). The methods for calculating the equivalent scattering coefficient $\mu_s'(\lambda_1)$and the absorption coefficient $\mu_a(\lambda_1)$ have been described above.

As described above, the measuring device and method according to the present embodiment allow precise measurement of the blood turbidity.

An embodiment has been described above, and the embodiment is presented by way of example and is not intended to limit the scope of the invention. The novel embodiment can be implemented in a variety of other forms, and a variety of omissions, replacements, and changes can be made to the embodiment to the extent that they do not depart from the substance of the invention. The embodiment and variations thereof are encompassed in not only the scope and substance of the invention but the invention described in the claims and the scope equivalent thereto.

Reference Sings list

1 Noninvasive measuring device
2 Light radiator
3 Optical intensity detector
4 Control unit

## Claims

**1.** A noninvasive measuring device comprising:

a light radiator that radiates light to a predetermined position on a subject;
optical intensity detectors that are disposed in positions above a blood vessel of the subject and detect a first optical intensity of light emitted from the subject in positions separate at predetermined intervals or in continuous positions from a position irradiated with the light from the light radiator;
optical intensity detectors that are disposed in positions not above the blood vessel of the subject and detect a second optical intensity of light emitted from the subject in positions separate at predetermined intervals or in continuous positions from the position irradiated with the light from the light radiator; and
a control unit that calculates a third optical intensity from blood in the blood vessel by using the first optical intensity and the second optical intensity.

**2.** The noninvasive measuring device according to claim 1,
wherein the control unit calculates the third optical intensity by using Numerical Formula 1 below,

[Numerical Formula 1]

$$R_{blood}(\rho) = \frac{(1-\beta)R_{on} - (1-\alpha)R_{off}}{\alpha - \beta}$$

where $R_{blood}$ (p) represents the third optical intensity, $R_{on}$ represents the first optical intensity, $R_{off}$ represents the second optical intensity, and $\alpha$ and $\beta$ represent values determined in advance.

**3.** The noninvasive measuring device according to claim 1 or 2, wherein the control unit calculates an attenuation coefficient from the third optical intensity.

**4.** The noninvasive measuring device according to claim 3, wherein the control unit calibrates the attenuation coefficient by multiplying the attenuation coefficient by a calibration coefficient.

**5.** The noninvasive measuring device according to claim 3 or 4,

wherein the light radiator radiates light having a first wavelength and light having a second wavelength, and

the control unit
calculates a first attenuation coefficient in accordance with which the light having the first wavelength is attenuated and a second attenuation coefficient in accordance with which the light having the second wavelength is attenuated, and
separates and calculates an equivalent scattering coefficient or an absorption coefficient for the blood from the first attenuation coefficient and the second attenuation coefficient.

**6.** A noninvasive measuring program that causes a computer in a device including a light radiator that radiates light to a predetermined position on a subject, optical intensity detectors that are disposed in positions above a blood vessel of the subject and detect a first optical intensity of light emitted from the subject in positions separate at predetermined intervals from a position irradiated with the light from the light radiator, and optical intensity detectors that are disposed in positions not above the blood vessel of the subject and detect a second optical intensity of light emitted from the subject in positions separate at predetermined intervals from the position irradiated with the light from the light radiator to carry out the process of calculating a third optical intensity from blood in the blood vessel by using the first optical intensity and the second optical intensity.

**7.** A noninvasive measuring method comprising:

radiating light to a predetermined position on a subject;
detecting a first optical intensity of light emitted from the subject in positions on a blood vessel of the subject and separate at predetermined intervals from a position irradiated with the light;
detecting a second optical intensity of light emitted from the subject in positions not on the blood vessel of the subject and separate at predetermined intervals from the position irradiated with the light; and
calculating a third optical intensity from blood in the blood vessel by using the first optical intensity and the second optical intensity.

FIG. 1

CONTROL UNIT
1
4
2
21
31a
31b
31c
31d
31e
31f
A
B
ρ1
ρ2
ρ3

FIG. 2

A
B
31c
31b
31a
21
2
31d
31e
31f
32f
32e
32d
32a
32b
32c
$\rho_1$
$\rho_1$
$\rho_2$
$\rho_2$
$\rho_3$
$\rho_3$

FIG. 3

2
3
ρ
21
22
A
B
C
E
D

FIG. 4

41
4
CPU
43
ROM
RAM
44
46
STORAGE
EXTERNAL I/F
47
42
2
LIGHT RADIATOR
OPTICAL INTENSITY DETECTOR
3

FIG. 5

$\ln\left\{\rho^2 \frac{R(\rho)}{s_0}\right\}$
*Slope is* $-\mu_{eff}$
ρ
$\frac{1}{\mu_{eff}} \ln\left(\frac{\mu_{eff}}{2\pi\mu_s'}\right)$

FIG. 6

STEP101
RADIATE LIGHT
STEP102
MEASURE OPTICAL INTENSITY
STEP103
CALCULATE OPTICAL INTENSITY FROM BLOOD
STEP104
CALCULATE ATTENUATION COEFFICIENT
STEP105
CALIBRATE ATTENUATION COEFFICIENT
STEP106
CALCULATE SCATTERING COEFFICIENT

## INTERNATIONAL SEARCH REPORT

International application No. PCT/JP2020/021723

A. CLASSIFICATION OF SUBJECT MATTER
Int.Cl. G01N21/49(2006.01)i, A61B5/1455(2006.01)i
FI: A61B5/1455, G01N21/49Z

According to International Patent Classification (IPC) or to both national classification and IPC

B. FIELDS SEARCHED

Minimum documentation searched (classification system followed by classification symbols)
Int.Cl. G01N21/49, A61B5/1455

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | JP 2015-123341 A (SEIKO EPSON CORPORATION) 06.07.2015 (2015-07-06), abstract, paragraphs [0058]-[0077], [0114], [0120], fig. 1, 5, 6 | 1, 6-7<br>3-5<br>2 |
| X<br>Y<br>A | US 2006/0129038 A1 (ZELENCHUK, A. R.) 15.06.2006 (2006-06-15), abstract, paragraphs [0004], [0057]-[0064], fig. 4A-6B | 1, 6-7<br>3-5<br>2 |
| Y | WO 2018/151150 A1 (MEDICAL PHOTONICS CO., LTD.) 23.08.2018 (2018-08-23), abstract, paragraphs [0029]-[0079], [0087]-[0092] | 3-5 |
| A | WO 2017/119130 A1 (MITSUBISHI CHEMICAL HOLDINGS CORPORATION) 13.07.2017 (2017-07-13), abstract, paragraphs [0031]-[0035], fig. 4, 6 | 1-7 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27.07.2020 | 04.08.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2020/021723

C (Continuation). DOCUMENTS CONSIDERED TO BE RELEVANT

| Category* | Citation of document, with indication, where appropriate, of the relevant passages | Relevant to claim No. |
|---|---|---|
| A | JP 2019-17956 A (ONO, Shoichi) 07.02.2019 (2019-02-07), abstract, paragraphs [0059]-[0086], fig. 1, 11-15 | 1-7 |
| A | JP 2018-29749 A (SEIKO EPSON CORPORATION) 01.03.2018 (2018-03-01), paragraphs [0040]-[0053], fig. 9 | 1-7 |

Form PCT/ISA/210 (second sheet) (January 2015)

**INTERNATIONAL SEARCH REPORT**
Information on patent family members

International application No.
PCT/JP2020/021723

| | | |
|---|---|---|
| JP 2015-123341 A | 06.07.2015 | US 2015/0182150 A1<br>abstract, paragraphs [0083]-[0102], [0141], [0147], fig. 1, 5, 6<br>EP 2888998 A2<br>CN 104739423 A |
| US 2006/0129038 A1 | 15.06.2006 | WO 2006/113748 A2 |
| WO 2018/151150 A1 | 23.08.2018 | (Family: none) |
| WO 2017/119130 A1 | 13.07.2017 | (Family: none) |
| JP 2019-17956 A | 07.02.2019 | (Family: none) |
| JP 2018-29749 A | 01.03.2018 | US 2018/0055428 A1<br>paragraphs [0060]-[0074], fig. 9<br>CN 107773249 A |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6241853 B


### Non-patent literature cited in the description


- **SHIMIZU KOICHI.** Analysis of Light Propagation in Biological Tissue. *Japanese Journal of Optics,* 2012, vol. 41 (8), 414-423